# EUROPEAN PATENT SPECIFICATION

(11) **EP 4 085 856 B1**
(45) Date of publication and mention of the grant of the patent: **11.12.2024**
(21) Application number: 20909843.3
(22) Date of filing: 09.11.2020
(51) Int. Cl.: A61B 17/70

(54) **MULTIFUNCTIONAL VERTEBRAL BODY FORMER**
MULTIFUNKTIONALER WIRBELKÖRPERBILDNER
DISPOSITIF MULTIFONCTIONNEL DE FORMATION DE CORPS VERTÉBRAL

(30) Priority: 30.12.2019 CN 201911402650
(43) Date of publication of application: 09.11.2022
(73) Proprietor: Ningbo Hicren Biotechnology Co., Ltd., Ningbo, Zhejiang 315336 (CN)
(72) Inventor: YU, Jie, Hangzhou Bay New District Ningbo, Zhejiang 315336 (CN); LI, Dong, Hangzhou Bay New District Ningbo, Zhejiang 315336 (CN); HU, Dong, Hangzhou Bay New District Ningbo, Zhejiang 315336 (CN); ZHANG, Pengyun, Hangzhou Bay New District Ningbo, Zhejiang 315336 (CN); LV, Shiwen, Hangzhou Bay New District Ningbo, Zhejiang 315336 (CN)
(74) Representative: Lindner Blaumeier Patent- und Rechtsanwälte
(86) International application number: PCT/CN2020/127601
(87) International publication number: WO 2021/135649

(56) References cited:
- CN-A- 110 897 696
- CN-U- 203 647 842
- CN-U- 204 699 234
- CN-U- 206 315 359
- CN-U- 206 315 359
- CN-U- 209 611 287
- CN-U- 209 611 287
- CN-U- 211 862 939
- US-A1- 2012 330 300
- US-A1- 2015 094 731

## Description

### BACKGROUND OF THE INVENTION

### 1. Field of the Invention

The invention relates to the field of medical instruments, and more particularly, to a multifunctional vertebral body former.

### 2. Description of the Related Art

Existing percutaneous kyphoplasty PKP is a procedure involves inserting an inflatable balloon into a collapsed vertebral body through percutaneous puncture, raising an endplate through the expansion of the balloon, restoring the height of the vertebral body, correcting the kyphosis, forming a cavity surrounded by a bone shell inside the vertebral body, and injecting high viscosity bone cement under a low pressure. In order to make the balloon enter the vertebral body smoothly, a working channel needs to be created with a puncture needle and a sleeve, then a working channel for the balloon before its expansion is drilled by using a bone drill, the balloon is sent into the vertebral body, and the balloon is withdrawn after a cavity surrounded by a bone shell is formed inside the vertebral body, the bone cement is finally injected. The operation procedures are quite complex. In addition, due to the structure of the vertebral body, in order to maintain the biomechanical force balance of the vertebral body, balloon expansion needs to be performed on both sides of each vertebral body and the bone cement needs to be injected therein. All operations need to be operated under X-ray monitoring. As a result, the more complex the operation, the longer time the operation, the greater damage to the doctor's health, and the higher the requirements for doctor's physical strength and professional skills; balloon expansion and injection of bone cement on both sides will cause great trauma to the patient and a higher chance of complications for the patient; moreover, it may increase an economic burden on the patient. All of the above-mentioned disadvantages are quite obvious in the case of lesions of multiple vertebral bodies.

Chinese Patent Application No. CN109431589A discloses an integrated and multifunctional vertebral body former, comprising a balloon assembly, an angled adjustment assembly and an angled cavity opening assembly, but does not disclose in detail the connection relationship and working modes of various components at a proximal end to achieve multiple functions.

Chinese Patent Application No. CN109745114A and CN 209 611 287 disclose a multifunctional vertebral body former, comprising a balloon assembly and a cavity opening assembly. An outer tube assembly in the cavity opening assembly comprises an outer tube and an outer tube handle. The outer tube has an axial rigidity capable of withstanding a trust load from the opening of cavity for a tissue without deforming. The outer tube handle is provided with a cavity interface for injection of bone cement. However, it does not disclose details about the connection relationship and working modes for various components at a proximal end to achieve multiple functions.

Therefore, there's an urgent need for a vertebral body former which is easy to operate and simple in structure, so as to achieve the versatility of the vertebral body former.

### SUMMARY OF THE INVENTION

In order to solve the above-mentioned disadvantages in the prior art, the present invention provides a vertebral body former which is simple in structure and easy to operate. Expansion of a bone drill and pre-expansion of a balloon catheter are integrated in one instrument. In this way, the operation is simplified, operation time is reduced, operation efficiency is improved. In addition, since the operation needs to be performed under X-ray monitoring, shortening the operation time helps to reduce the damage to a doctor's health, and to reduce requirements for the doctor's physical strength and operational requirements.

For achieving the above-mentioned purposes, the invention adopts the following technical solution:
a multifunctional vertebral body former, comprising:
a balloon structure comprising a balloon outer tube, a balloon handle and a balloon; an outer surface of a proximal end of the balloon outer tube is provided with, in a sequence from inside to outside, a balloon handle fitting, the balloon handle and a balloon delivery snap from the inside out, and all of those components are fixedly connected with each other; wherein the balloon handle is connected with the balloon delivery snap by a snap in a rotationally fixed manner; the balloon outer tube is fixedly connected with a distal end of the balloon , the balloon is sealed at distal end thereof;
a conveying tube structure, comprising a shape memory alloy tube, a slider, and an outer tube adjusting handle, wherein the shape memory alloy tube is sleeved on the balloon outer tube and an exterior of the balloon, the shape memory alloy tube is formed integrally with the slider by injection molding; a proximal end of the slider is detachably connected with the balloon deliver snap through a first clamping lug; the slider is provided with a threaded outer surface, the outer tube adjusting handle having an internal thread structure is sleeved on the outer surface of the slider, the outer tube adjusting handle (22) is operatively rotated to drive the slider to move back and forth, so that a protruding amount of a distal end of the shape memory alloy tube relative to a distal end of the outer tube, and a protruding amount of a distal end of the balloon relative to the distal end of the shape memory alloy tube are adjustably controlled.

Preferably, the balloon structure further comprises a balloon deliver snap fitting sleeved outside of the balloon delivery snap, a second clamping lug at a distal end of the balloon delivery snap is inserted into a sliding chute of an inner surface of the balloon deliver snap fitting, a distal end of the sliding chute is provided with a slot; a distal end of the balloon deliver snap fitting is detachably connected with a limiting member sleeved outside of the slider by a snap; an inner rounded edge at a distal end of the limiting member extends a plurality of arc-shaped portions which are not connected with each other along an outer surface of the slider in a direction towards the distal end, a distal end of each of the plurality of arc-shaped portions are fixed to a proximal end of an end cap by a cylindrical pin, the end cap is match with and locked to a sleeve handle by a locking ring; the outer tube adjusting handle is sleeved outside of the arc-shaped portions, the threaded structure on the outer surface of the slider extends beyond a gap of the arc-shaped portions, and matches with the internal threaded structure of the outer tube adjusting handle.

Preferably, the locking ring is sleeved on an area external to a distal end of the end cap, a top block is sleeved between the distal end of the end cap and the locking ring , and is in threaded connection with the distal end of the end cap; a proximal end of the locking ring is fixed to the top block through the end cap, the proximal end of the locking ring can rotate about the end cap, and a distal end of the locking ring is fixed with the sleeve handle by the snap.

Preferably, the outer tube is sleeved on the exterior of the shape memory alloy tube, the outer tube and the end cap are integrally injection molded.

Since the outer tube has certain rigidity, it facilitates a distal end of the vertebral body former to enter into a specific position between vertebral bodies. When the distal end of the released shape memory alloy tube has a certain bending angle, it can be withdrawn into the outer tube before being withdrawn into the sleeve.

Preferably, a proximal end of the balloon handle extends a protrusion relative to a proximal end of the balloon handle fitting and a proximal end of the balloon delivery snap, a proximal end surface of the protrusion is closed to form a cavity, a side wall of the protrusion is provided with a cavity interface; the balloon outer tube is in communication with the outside through the protrusion.

Furthermore, the outside of the proximal end surface of the protrusion is connected to a supporting wire seat through a screw thread, the supporting wire seat is fixedly connected with a proximal end of the supporting wire, the supporting wire is inserted into a pore channel at the proximal end surface of the protrusion, the balloon outer tube and the balloon in sequence, and finally, a distal end surface of the supporting wire is in contact with the distal end of the balloon.

The supporting wire is made of a plastic material, so when the shape memory alloy tube has a certain bending angle, a force on the supporting wire is applied to make it bent, so that the distal end of the whole balloon structure is bent, and the bending angle is equal to that of the shape memory allot tube.

Preferably, a balloon inner tube is bonded to the balloon handle with the medical grade glue; the balloon handle fitting is bonded to the balloon handle with medical grade glue.

Preferably, a balloon inner tube is further provided or not provided between the balloon outer tube and the supporting wire, a proximal end of the balloon inner tube is fixed to the pore channel on the proximal end surface of the protrusion, a distal end of the balloon inner tube is contacted with the distal end of the balloon.

Furthermore, the balloon inner tube is not provided, the balloon outer tube is inserted from the proximal end of the balloon into the distal end of the balloon, both the proximal end and the distal end of the balloon are welded to the balloon outer tube by sweat soldering or laser welding; an opening is provided on the balloon outer tube in an interior of the balloon; the balloon outer tube at the distal end of the balloon is sealed with UV adhesives or PU rods.

Furthermore, in an embodiment where the balloon inner tube is provided, the distal end of the balloon inner tube is welded to the distal end of the balloon by sweat soldering or laser welding, and they are sealed in the balloon inner tube by the UV adhesives, and the supporting wire is in contact with the UV adhesives.

Furthermore, in an embodiment where the balloon inner tube is provided, the distal end of the balloon is welded to the balloon inner tube through a transition tube by sweat soldering or laser welding; after a proximal end of a plug is welded to the distal end of the supporting wire by laser welding, the proximal end of a plug and the distal end of the balloon are bonded to and sealed with the medical grade glue.

Preferably, a reinforcing tube is also sleeved on an outer surface of the balloon outer tube, and the reinforcing tube is bonded to the balloon outer tube with the medical grade glue, and the reinforcing tube and the balloon handle fitting are integrally injection molded.

The balloon outer tube is made of a PU material, so it is relatively softer. The reinforcing tube functions as: (1) protecting the balloon outer tube; and (2) making it easier to feed the balloon due to the rigidity of the reinforcing tube.

Preferably, a section of a curved portion is provided at the distal end of the shape memory alloy tube; the balloon is located at an interior of the shape memory alloy tube in its initial state. In this way, the distal end of the shape memory alloy tube is sealed, to some extent, so that cancellous bone inside the vertebral body can be prevented from entering the shape memory alloy tube during the process of vertebral expansion. The vertebral expansion herein refers to a process in which a head end of the shape memory alloy tube squeezes the cancellous bone inside the vertebral body to form a balloon passage.

By adopting the above-mentioned technical solutions, the present invention has the beneficial effects over the prior art:
1. the multifunctional vertebral body former provided in the present application combines functions of expansion of a bone drill and pre-expansion of a balloon catheter, and filling of bone cement, so that the number of surgical instruments is reduced, surgical procedures are simplified, operation time is saved, and economic burden on a patient is also reduced;
2. the shape memory alloy tube is delivered by using a threaded structure, ensuring a higher adjustment precision and a safer operation;
3. the shape memory alloy tube can directly reach the opposite side of the vertebral body from a side of the vertebral body which has been punctured at a bending angle, allowing to reduce the operation procedures and to reduce stress on the patient's body; in this way, only one puncture is required to finish the following procedures: the vertebral expansion (i.e., forming the balloon passage), balloon pre-expansion (i.e., creating a cavity filled with the bone cement), and injection of the bone cement is completed by directly connecting to a bone cement injection kit, and all of those procedures are convenient to operate.

### BRIEF DESCRIPTION OF THE DRAWINGS

Figure 1 is a schematic diagram showing a structure of a multifunctional vertebral body former according to the present invention;
Figure 2a is a cross-sectional view of a multifunctional vertebral body former at a first angle according to the present invention;
Figure 2b is a schematic diagram showing a structure of region A in Figure 2a;
Figure 3a is a cross-sectional view of a multifunctional vertebral body former at a second angle according to the present invention;
Figure 3b is a schematic diagram showing a structure of region B in Figure 2a;
Figure 4 is a cross-sectional view of the relative positional relationship of components at a proximal end of a multifunctional vertebral body former according to the present invention;
Figure 5 is a schematic diagram showing a connection status between a balloon delivery snap and a slider of a multifunctional vertebral body former according to the present invention;
Figure 6 is a schematic diagram showing a structure of the slider of a multifunctional vertebral body former according to the present invention;
Figure 7 is a schematic diagram showing a structure of a multifunctional vertebral body former after being inserted into a sleeve according to the present invention;
Figure 8a is a schematic diagram showing a position of the snap before a balloon of a multifunctional vertebral body former is pre-expanded according to the present invention;
Figure 8b is a schematic diagram showing a position of the snap after a balloon of a multifunctional vertebral body former is pre-expanded according to the present invention;
Figure 9 is a schematic diagram of a balloon delivery nap fitting of a multifunctional vertebral body former according to the present invention;
Figure 10a is a schematic diagram of a method for creating a balloon passage in example 2 of a multifunctional vertebral body former according to the present invention;
Figure 10b is a schematic diagram of a second method for creating a balloon passage in example 3 of a multifunctional vertebral body former according to the present invention;
Figure 11a is a schematic diagram of a working mode of balloon pre-expansion in example 2 of a multifunctional vertebral body former according to the present invention;
Figure 11b is a schematic diagram of a working mode of balloon pre-expansion in example 3 of a multifunctional vertebral body former according to the present invention;
Figure 12a is a schematic diagram of a working mode of pulling out a balloon structure from example 2 of a multifunctional vertebral body former according to the present invention;
Figure 12b is a schematic diagram of a working mode of pulling out a balloon structure from example 3 of a multifunctional vertebral body former according to the present invention;
Figure 13a is a cross-sectional diagram of the relative position between a balloon delivery snap and a limiting member before the balloon structure is pulled out by a multifunctional vertebral body former according to the present invention;
Figure 13b is a cross-sectional diagram of the relative position between a balloon delivery snap and a limiting member after the balloon structure is pulled out from a multifunctional vertebral body former according to the present invention;
Figure 14 is a schematic diagram of a balloon structure of a multifunctional vertebral body former according to the present invention;
Figure 15 is a schematic diagram showing a structure of a multifunctional vertebral body former from which a balloon structure is pulled out according to the present invention;
Figure 16a is a schematic diagram showing a processing mode of a distal end of a first balloon of a multifunctional vertebral body former according to the present invention;
Figure 16b is a schematic diagram showing a processing mode of a distal end of a second balloon of a multifunctional vertebral body former according to the present invention;
Figure 16c is a schematic diagram showing a processing mode of a distal end of a third balloon of a multifunctional vertebral body former according to the present invention;
Figure 16d is a schematic diagram showing a processing mode of a distal end of a fourth balloon of a multifunctional vertebral body former according to the present invention;
Figure 16e is a schematic diagram showing a processing mode of a distal end of a fifth balloon of a multifunctional vertebral body former according to the present invention;
Figure 16f is a schematic diagram showing a processing mode of a distal end of a six balloon of a multifunctional vertebral body former according to the present invention; and
Figure 17 is a schematic diagram showing a structure of an end cap of a multifunctional vertebral body former according to the present invention.

Reference numerals in the accompanying drawings:
1-balloon structure, 11-balloon handle, 111-protrusion, 112- cavity interface, 113-supporting wire seat, 114-pore channel, 12-balloon handle fitting, 121-reinforcing tube, 13-balloon delivery snap, 131-second clamping lug; 132-fourth clamping lug, 14-balloon delivery snap fitting, 141-chute, 142-slot, 143-third clamping lug, 15-balloon, 151-plug, 152-transition tube, 153- UV adhesive, 154- PU rod, 16- balloon outer tube, 161-outer tube opening, 17-balloon inner tube, 18-supporting wire,
2-conveying tube structure, 21-shape memory alloy tube, 22-outer tube adjusting handle, 23-slider, 231- first clamping lug, 24-limiting member, 241-limiting slot, 242-arc-shaped portion, 25-end cap, 251-cylinderical pin, 26-locking ring, 261-top block, 27-curved portion, 28-outer tube,
3-sleeve structure, 31-sleeve handle, 32-sleeve.

### DETAILED DESCRIPTION

For better understanding of the invention, the present invention will be described clearly and fully hereinafter with reference to the particular embodiments, but the invention is not limited thereto.

In the following examples, a proximal end refers to a direction close to an operator, and a distal end refers to a direction close to a patient; clockwise refers to the clockwise direction relative to the operator, and counterclockwise refers to the counterclockwise direction relative to the operator.

### Example 1

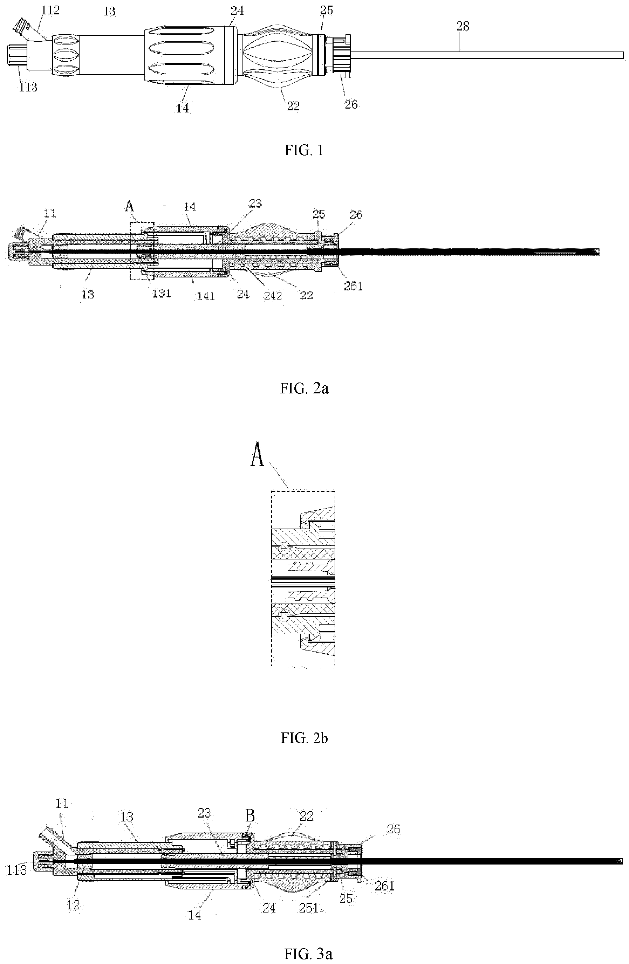

The embodiment of the invention provides a multifunctional vertebral body former, as shown in Figures1, 2a-2b and 3a-3b, comprising:
a balloon structure 1 comprising a balloon outer tube 16, a balloon handle 11 and a balloon 15; an outer surface of a proximal end of the balloon outer tube 16 is provided with, in a sequence from inside to outside, a balloon handle fitting 12, the balloon handle 11 and a balloon delivery snap 13; wherein the balloon handle 11 is connected with the balloon delivery snap 13 by a fourth clamping lug 132 on the balloon delivery snap 13 and a slot on the balloon handle 11 in a rotationally fixed manner; the balloon handle fitting 12 is fixedly connected with the balloon handle 11; a distal end of the balloon outer tube 16 is fixedly connected with the balloon 15, and the balloon 15 is sealed at a distal end thereof;
a conveying tube structure 2, comprising a shape memory alloy tube 21, a slider 23, and an outer tube adjusting handle 22, wherein the shape memory alloy tube 21 is sleeved on the balloon outer tube 16 and an exterior of the balloon 15, the shape memory alloy tube 21 is formed integrally with the slider 23 by injection molding; a proximal end of the slider 23 is detachably connected with the balloon deliver snap 13 through a first clamping lug 231; the slider 23 is provided with a threaded outer surface, the outer tube adjusting handle 22 having an internal thread structure is sleeved on the outer surface of the slider 23, the outer tube adjusting handle 22 is operatively rotated to drive the slider 23 to move back and forth, so that a protruding amount of a distal end of the shape memory alloy tube 21 relative to a distal end of the outer tube 28, and a protruding amount of a distal end of the balloon 15 relative to the distal end of the shape memory alloy tube 21 are adjustably controlled.

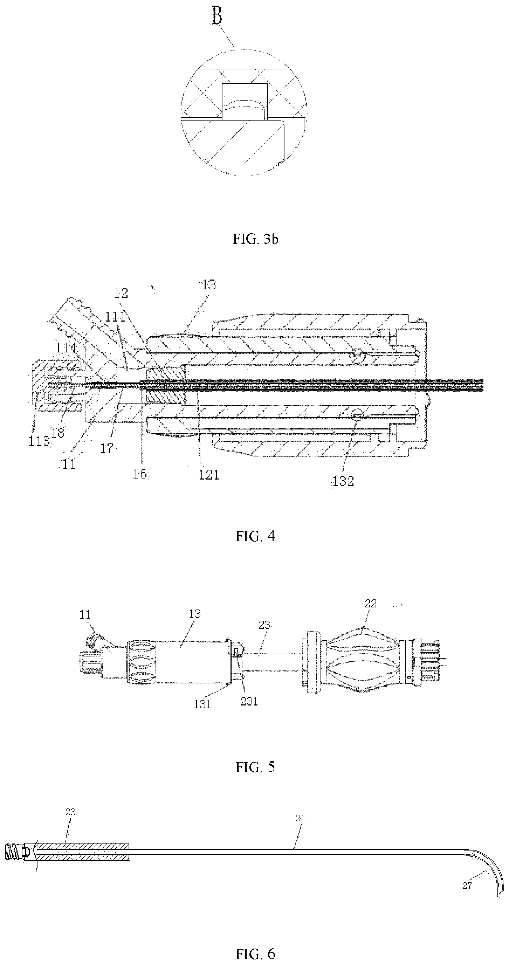

In this embodiment, as shown in Figures 2a-2b, 3a-3b, the balloon structure 1 further comprises a balloon deliver snap fitting 14 sleeved outside of the balloon delivery snap 13, a second clamping lug 131 at a distal end of the balloon delivery snap 13 is inserted into a sliding chute 141 of an inner surface of the balloon deliver snap fitting 14, a distal end of the sliding chute 141 is provided with a slot 142; a distal end of the balloon deliver snap fitting 14 is detachably connected with a limiting member 24 sleeved outside of the slider 23 by a snap; an inner rounded edge at a distal end of the limiting member 24 extends a plurality of arc-shaped portions 242 which are not connected with each other along an outer surface of the slider 23 in a direction towards the distal end, a distal end of each of the plurality of arc-shaped portions 242 are fixed to a proximal end of an end cap 25 by a cylindrical pin 252, the end cap 25 is match with and locked to a sleeve handle 31 by a locking ring 26; the outer tube adjusting handle 22 is sleeved outside of the arc-shaped portions 242, the threaded structure on the outer surface of the slider 23 extends beyond a gap of the arc-shaped portions 242, and matches with the internal threaded structure of the outer tube adjusting handle 22.

In this embodiment, the locking ring 26 is sleeved on an area external to a distal end of the end cap 25, a top block 261 is sleeved between the distal end of the end cap 25 and the locking ring 26, and is in threaded connection with the distal end of the end cap 25; a proximal end of the locking ring 26 is fixed to the top block 261 through the end cap 25, the proximal end of the locking ring 26 can rotate about the end cap 25, and a distal end of the locking ring 26 is fixed with the sleeve handle 31 by the snap.

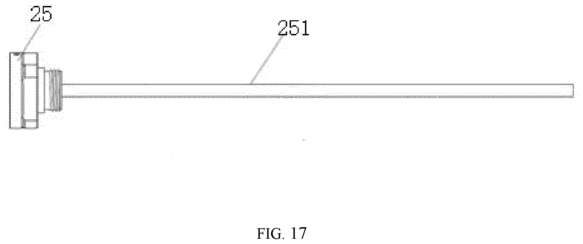

In this embodiment, as shown in Figures 1, 17, the outer tube 28 is sleeved on the exterior of the shape memory alloy tube 21, the outer tube 28 is formed integrally with the end cap 25 by injection molding. Since the outer tube 28 has certain rigidity, it facilitates a distal end of the vertebral body former to enter into a specific position between vertebral bodies. When the distal end of the released shape memory alloy tube 21 has a certain bending angle, it can be withdrawn into the outer tube 28 before being withdrawn into the sleeve 32.

In this embodiment, as shown in Figure 4, a proximal end of the balloon handle 11 extends a protrusion 111 relative to a proximal end of the balloon handle fitting 12 and a proximal end of the balloon delivery snap 13, a proximal end surface of the protrusion 111 is closed to form a cavity, a side wall of the protrusion 111 is provided with a cavity interface 112; the balloon outer tube 16 is in communication with the outside through the protrusion 111.

In this embodiment, as shown in Figures 2a, 3a, 4, 16a-16f, the outside of the proximal end surface of the protrusion 111 is connected to a supporting wire seat 113 through a screw thread, the supporting wire seat 113 is fixedly connected with a proximal end of the supporting wire 18, the supporting wire 18 is inserted into a pore channel 114 at the proximal end surface of the protrusion 111, the balloon outer tube 16 and the balloon 15 in sequence, and finally, a distal end surface of the supporting wire 18 is in contact with the distal end of the balloon 15; when the distal end of the supporting wire 18 is not provided with the plug 151 welded thereto, it can be separated from the supporting seat by rotating the supporting wire seat 113, and then the whole supporting wire 18 is pulled out.

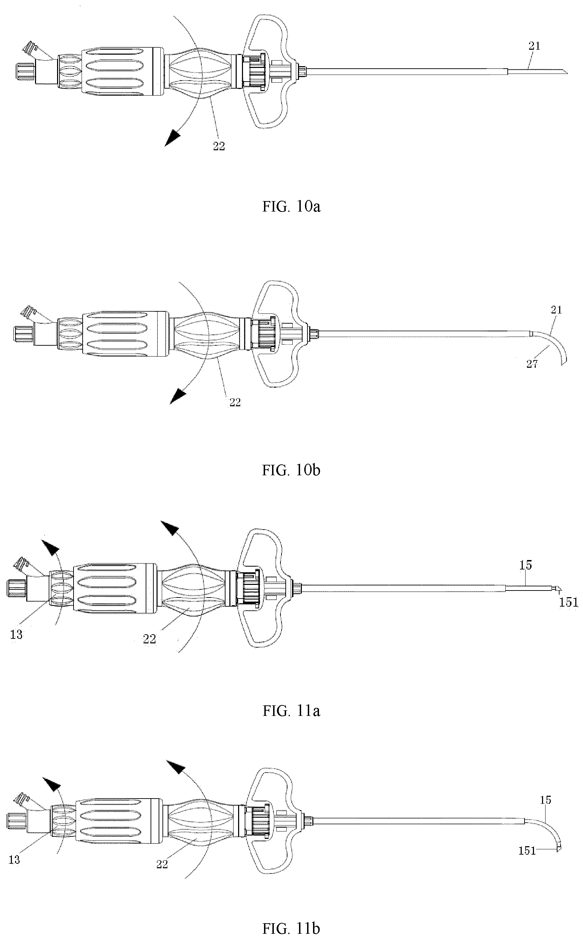

In an embodiment of the invention, in Figure 10b, a section of a curved portion 27 is provided at the distal end of the shape memory alloy tube 21; the balloon 15 is located at an interior of the shape memory alloy tube 21 in its initial state. In this way, the distal end of the shape memory alloy tube 21 is sealed, to some extent, so that cancellous bone inside the vertebral body can be prevented from entering the shape memory alloy tube 21 during the process of vertebral expansion. The vertebral expansion herein refers to a process in which a head end of the shape memory alloy tube squeezes the cancellous bone inside the vertebral body to form a balloon passage; preferably, the shape memory alloy tube itself is a memory metal, and the shape memory alloy tube can form a bending angle of 90° after heat treatment through a shaping angle designed on a shaping die; the aim of the heat treatment is to reduce its Af point (the temperature when it returns to the cornered state) to obtain superelasticity, so the shape memory alloy tube can return to the cornered state by virtue of its own memory properties and the superelasticity obtained from the heat treatment after extending beyond the outer tube; furthermore, in this embodiment, after heat-setting the shape memory alloy tube by the heat treatment, the Af point is controlled at a temperature of about 20 °C, when a temperature is higher than the Af point, the shape memory alloy tube can quickly return to the cornered state. When the balloon passage is established, the shape memory alloy tube enters the inside of the vertebral body, and the temperature inside the human body is about 37°C, so when the shape memory alloy tube comes out of a sleeve, it will create the balloon passage inside the vertebral body at a determined bending angel, so that the shape memory alloy tube can reach the opposite side of the vertebral body from a side of the vertebral body which has been punctured through the curved portion.

In this embodiment, the supporting wire 18 is made of semi-rigid material, which provides sufficient rigidity to the distal end of the balloon structure 1 so that it can enter into an area between the vertebral bodies; the distal end of the shape memory alloy tube 21 has a curved portion 27. As shown in Figure 10b, when the shape memory alloy tube 21 exhibits a certain bending angle, a force is applied to the supporting wire 18 to make it bent, so that the distal end of the entire balloon structure 1 becomes bent, and the bending angle is the same as that of the shape memory alloy tube 21, as shown in Figure 11b.

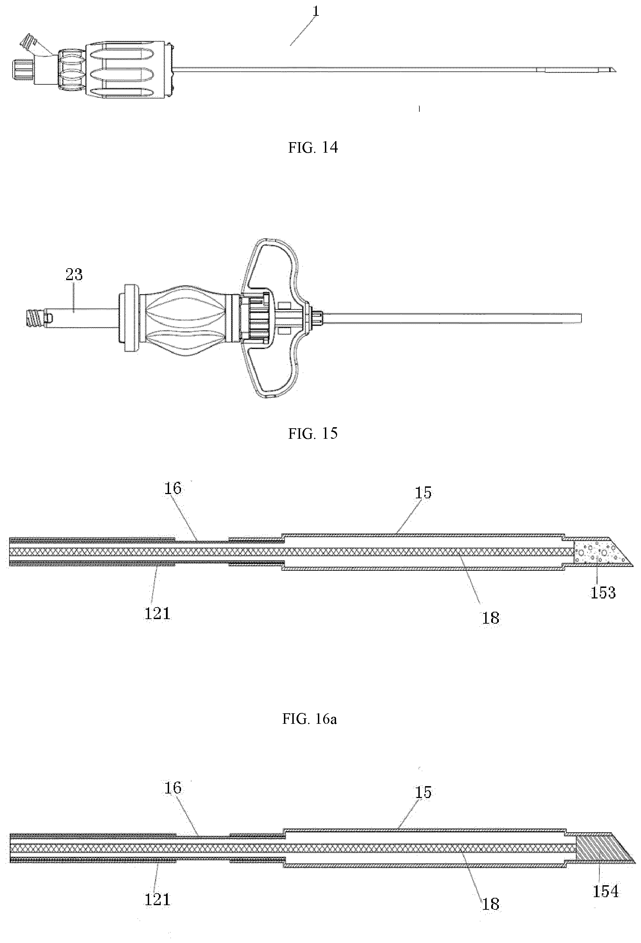

In this embodiment, as shown in Figures 4, 16a-16b, a reinforcing tube 121 is also sleeved on an outer surface of the balloon outer tube 16, and the reinforcing tube 121 is bonded with the balloon outer tube 16 with the medical grade glue, and the reinforcing tube 121 and the balloon handle fitting 12 are integrally injection molded.

In this embodiment, as shown in Figure 16a, the distal end of the balloon outer tube 16 is welded to the proximal end of the balloon 15 by sweat soldering or laser welding, and the distal end of the balloon 15 is sealed with the UV adhesives 153.

In an embodiment of the invention, as shown in Figure 16b, the distal end of the balloon outer tube 16 is welded to the proximal end of the balloon 15 by sweat soldering or laser welding, and the distal end of the balloon 15 is sealed with the balloon through PU rods 154 by sweat soldering or laser welding.

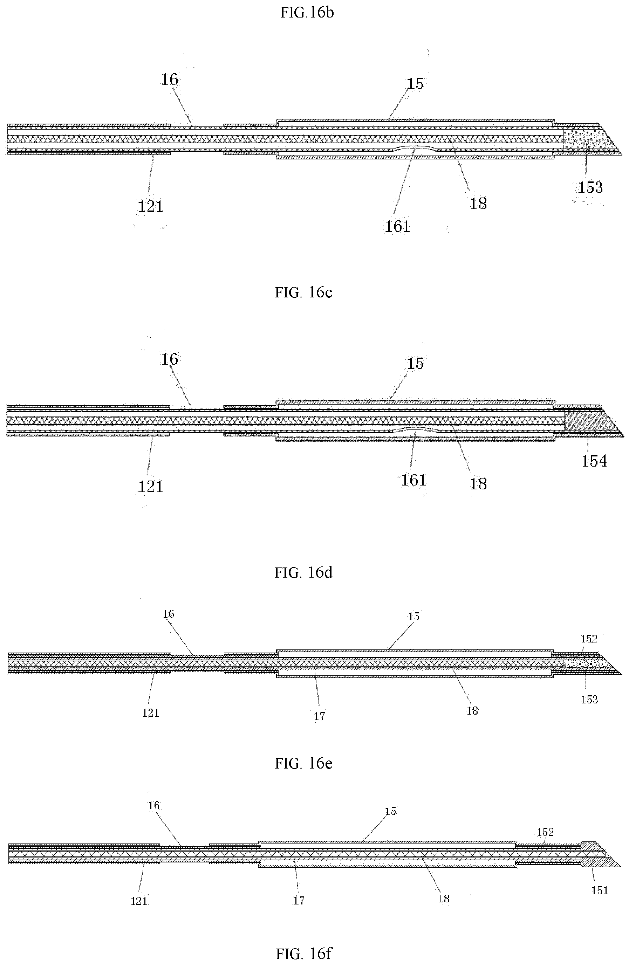

In an embodiment of the invention, as shown in Figure 16c, the balloon outer tube 16 is directly inserted from the proximal end of the balloon 15 into the distal end of the balloon 15, both the proximal end and the distal end of the balloon 15 are welded to the balloon outer tube 16 by sweat soldering or laser welding; an outer tube opening 161 is provided on the balloon outer tube 16 in an interior of the balloon 15; the balloon outer tube 16 at the distal end of the balloon 15 is sealed with UV adhesives 153.

In an embodiment of the invention, as shown in Figure 16d, the balloon outer tube 16 is directly inserted from the proximal end of the balloon 15 into the distal end of the balloon 15, both the proximal end and the distal end of the balloon 15 are welded to the balloon outer tube 16 by sweat soldering or laser welding; an outer tube opening 161 is provided on the balloon outer tube 16 in an interior of the balloon 15; the balloon outer tube 16 at the distal end of the balloon 15 is sealed with the balloon through the PU rods 154 by sweat soldering or laser welding.

In an embodiment of the invention, as shown in Figures 16e-16f, the distal end of the balloon outer tube 16 is welded to the proximal end of the balloon 15 by sweat soldering or laser welding; a balloon inner tube 17 is provided between the balloon outer tube 16 and the supporting wire 18, a proximal end of the balloon inner tube 17 is fixed to the pore channel 114 at a surface of the proximal end of the protrusion 111, a distal end of the balloon inner tube 17 is fixedly connected with the distal end of the balloon 15.

In an embodiment of the invention, as shown in Figure 16e, the distal end of the balloon 15 is welded to the balloon inner tube 17 through a transition tube 152 by sweat soldering or laser welding; the balloon inner tube 17 is sealed with the UV adhesives 153, and the distal end of the supporting wire 18 bears against the UV adhesives 153.

In an embodiment of the invention, as shown in Figure 16f, the distal end of the balloon 15 is welded to the balloon inner tube 17 through the transition tube 152 by sweat soldering or laser welding; after a proximal end of a plug 151 is welded to the distal end of the supporting wire 18 by laser welding, the proximal end surface of a plug 151 and the distal end surface of the balloon 15 are bonded to and sealed with the medical grade glue.

### Example 2

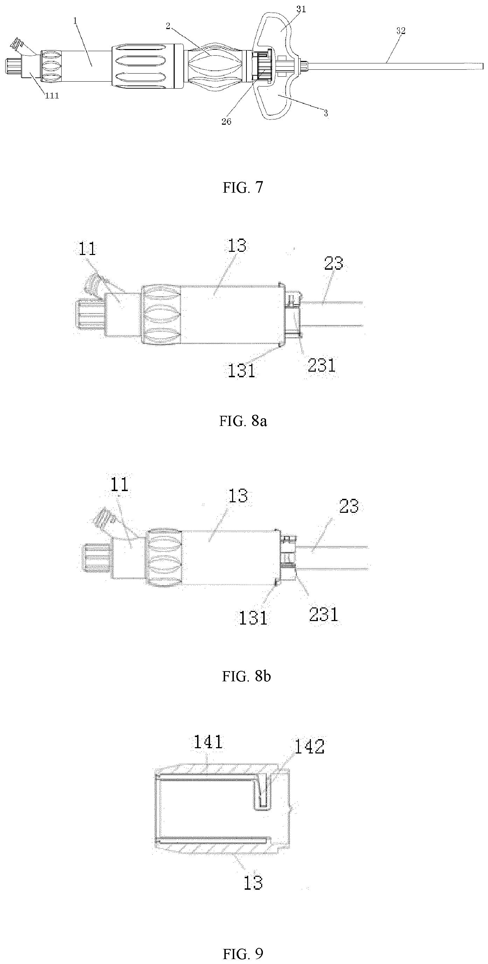

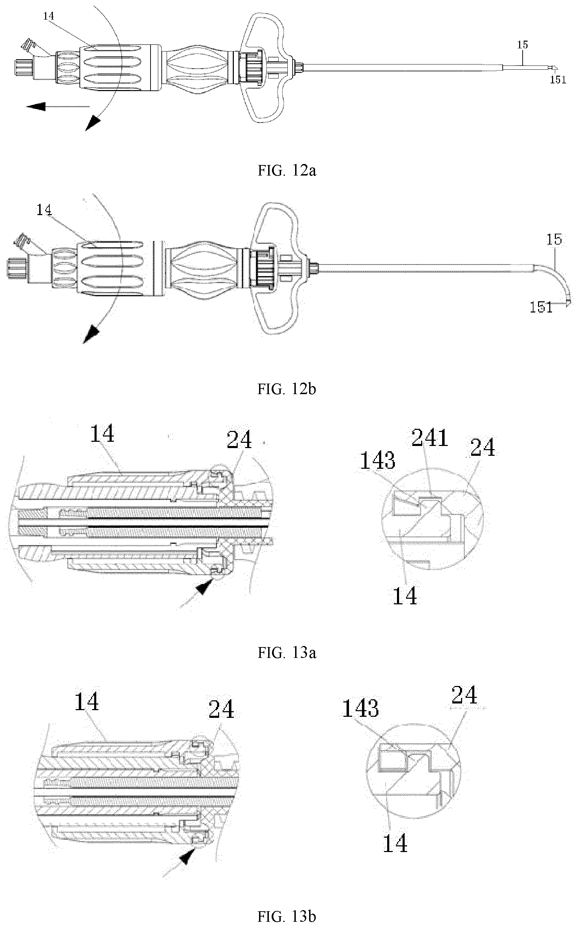

This embodiment provides a working principle of the multifunctional vertebral body former as described in Example 1.
(1) before it works, as shown in Figures 2a, 3a, 5, 14-15, the conveying tube structure 2 is connected to an area outside of the balloon structure 1 by a snap, the first clamping lug 231 on the slider 23 is clamped on the slot at the distal end of the balloon delivery snap 13, the third clamping lug 143 on the balloon delivery snap fitting 14 is clamped on the limiting slot 241 on the limiting member 24; as shown in Figures 1 and 5, after a working channel is established, the multifunctional vertebral body former provided in Example 1 is inserted into the sleeve structure 3, rotating the locking ring 26 to make the multifunctional vertebral body former fixed to the sleeve handle 31 on the sleeve structure 3;
(2) as shown in Figure 10a, rotating the outer tube adjusting handle 22 clockwise, and driving the balloon structure 1 and the slider 23 to move forward through the thread transmission of the outer tube adjusting handle 22 and the slider 23, so that the balloon 15 follows the shape memory alloy tube 21 to extend into the interior of the vertebral body until the shape memory alloy tube 21 extends to a preset position, so as to achieve vertebral expansion and establish a balloon passage; as shown in Figure 9, after the balloon passage is established, the second clamping lug 131 on the balloon delivery snap 13 is located at a bottom of the chute 141 of the balloon delivery snap fitting 14;
(3) as shown in Figures 8a-8b,Figure 9, and Figure 11, rotating the balloon delivery snap 13 counterclockwise to make the first clamping lug 231 on the slider 23 completely free from the restriction of the slot at the distal end of the balloon delivery snap 13, and thus to make the slider 23 completely free from the restriction of the balloon delivery snap 13, and rotating the second clamping lug 131 into the slot 142 at a bottom of the chute 141 of the balloon delivery snap fitting 14; then rotating the outer tube adjusting handle 22 counterclockwise so that the shape memory alloy tube 21 can be withdrawn into the outer tube 28, and the balloon 15 is completely placed into the well-established balloon passage;
(4) a connection to a pressure pump is established through the cavity interface 112 on the balloon handle 11, and contrast agent is injected into the balloon 15 to reset the vertebral body for compression fractures and create a cavity filled with bone cement to achieve unilateral balloon pre-expansion; after the unilateral balloon pre-expansion is completed, the contrast agent injected into the balloon 15 is withdrawn by the pressure pump;
(5) the vertebral body is punctured at the other side using the same other surgical device, so as to achieve balloon pre-expansion at the other side of the vertebral body, after the balloon pre-expansion is completed, the contrast agent injected into the balloon 15 is withdrawn by the pressure pump;
(6) as shown in Figures 13a-13b and 12a, rotating the balloon delivery snap fitting 14 clockwise to make the two third clamping lugs 143 on the balloon delivery snap fitting 14 free from the restriction of the limiting member 24, and then pulling out the balloon structure as shown in Figure 14;
(7) after the bone cement injection kit is connected to a joint on the slider 23 in a structure as shown in Figure 15, the bone cement is injected into the established bone cement filling cavity, and finally the sleeve is withdrawn and the operation is completed.

### Example 3

This example provides a working principle of the multifunctional vertebral body former as described in Example 1.
(1) before it works, as shown in Figures 2a, 3a, 5, 14-15, the conveying tube structure 2 is connected to an area outside of the balloon structure 1 by a snap, the first clamping lug 231 on the slider 23 is clamped on the slot at the distal end of the balloon delivery snap 13, the third clamping lug 143 on the balloon delivery snap fitting 14 is clamped on the limiting slot 241 on the limiting member 24; as shown in Figures 1 and 5, after a working channel is established, the multifunctional vertebral body former provided in Example 1 is inserted into the sleeve structure 3, rotating the locking ring 26 to make the multifunctional vertebral body former fixed to the sleeve handle 31 on the sleeve structure 3;
(2) as shown in Figure 10b, rotating the outer tube adjusting handle 22 clockwise, and driving the balloon structure 1 and the slider 23 to move forward through the thread transmission of the outer tube adjusting handle 22 and the slider 23, so that the balloon 15 follows the shape memory alloy tube 21 to extend into the interior of the vertebral body until the shape memory alloy tube 21 extends to a preset position, so as to achieve vertebral expansion and establish a balloon passage, the shape memory alloy tube 21 can directly reach the opposite side of the vertebral body by puncturing at one side of the vertebral body through the curved portion 27; as shown in Figure 9, after the balloon passage is established, the second clamping lug 131 on the balloon delivery snap 13 is located at a bottom of the chute 141 of the balloon delivery snap fitting 14;
(3) as shown in Figures 8a-8b, Figure 9, and Figure 11b, rotating the balloon delivery snap 13 counterclockwise to make the first clamping lug 231 on the slider 23 completely free from the restriction of the slot at the distal end of the balloon delivery snap 13, and thus to make the slider 23 completely free from the restriction of the balloon delivery snap 13, and rotating the second clamping lug 131 into the slot 142 at a bottom of the chute 141 of the balloon delivery snap fitting 14, the balloon delivery snap 13 is locked with the balloon delivery snap fitting 14; then rotating the outer tube adjusting handle 22 counterclockwise so that the shape memory alloy tube 21 can be withdrawn into the outer tube 28, and the balloon 15 is completely placed into the well-established balloon passage;
(4) a connection to a pressure pump is established through the cavity interface 112 on the balloon handle 11, and contrast agent is injected into the balloon 15 to reset the vertebral body for compression fractures and create a cavity filled with bone cement to achieve unilateral balloon pre-expansion; after the unilateral balloon pre-expansion is completed, the contrast agent injected into the balloon 15 is withdrawn by the pressure pump;
(5) as shown in Figures 12a and 13a-13b, rotating the balloon delivery snap fitting 14 clockwise to make the two third clamping lugs 143 on the balloon delivery snap fitting 14 free from the restriction of the limiting member 24, and then pulling out the balloon structure as shown in Figure 14;
(6) after the bone cement injection kit is connected to a joint on the slider 23 in a structure as shown in Figure 15, the bone cement is injected into the established bone cement filling cavity, and finally the sleeve is withdrawn and the operation is completed.

## Claims

1. A multifunctional vertebral body former, comprising:
a balloon structure (1) comprising a balloon outer tube (16), a balloon handle (11) and a balloon (15); an outer surface of a proximal end of the balloon outer tube (16) is provided with, in a sequence from inside to outside, a balloon handle fitting (12), the balloon handle (11) and a balloon delivery snap (13) ; wherein the balloon handle (11) is connected with the balloon delivery snap (13) by a snap in a rotationally fixed manner, the balloon handle fitting (12) is fixedly connected with the balloon handle (11); a distal end of the balloon outer tube (16) is fixedly connected with the balloon (15), the balloon (15) is sealed at a distal end thereof;
a conveying tube structure (2), comprising a shape memory alloy tube (21), a slider (23), and an outer tube adjusting handle (22), wherein the shape memory alloy tube (21) is sleeved on the balloon outer tube (16) and an exterior of the balloon (15), the shape memory alloy tube (21) is formed integrally with the slider (13) by injection molding ; a proximal end of the slider (23) is detachably connected with the balloon deliver snap (13) through a first clamping lug (231); the slider (23) is provided with a threaded outer surface, the outer tube adjusting handle (22) having an internal thread structure is sleeved on the outer surface of the slider (23), wherein the outer tube adjusting handle (22) is operatively rotated to drive the slider (23) to move back and forth, so that a protruding amount of a distal end of the shape memory alloy tube (21) relative to a distal end of the outer tube (28), and a protruding amount of a distal end of the balloon (15) relative to the distal end of the shape memory alloy tube (21) are adjustably controlled.

2. The multifunctional vertebral body former of claim 1, wherein the balloon structure (1) further comprises a balloon deliver snap fitting (14) sleeved outside of the balloon delivery snap (13), a second clamping lug (131) at a distal end of the balloon delivery snap (13) is inserted into a sliding chute (141) of an inner surface of the balloon deliver snap fitting (14), a distal end of the sliding chute (141) is provided with a slot (142); a distal end of the balloon deliver snap fitting (14) is detachably connected with a limiting member (24) sleeved outside of the slider (23) by a snap; an inner rounded edge at a distal end of the limiting member (24) extends a plurality of arc-shaped portions (242) which are not connected with each other along an outer surface of the slider (23) in a direction towards the distal end, a distal end of each of the plurality of arc-shaped portions (242) are fixed to a proximal end of an end cap (25) by a cylindrical pin (251), the end cap (25) is match with and locked to a sleeve handle (31) by a locking ring (26); the outer tube adjusting handle (22) is sleeved outside of the arc-shaped portions (242), the threaded structure on the outer surface of the slider (23) extends beyond a gap of the arc-shaped portions (242), and matches with the internal threaded structure of the outer tube adjusting handle (22).

3. The multifunctional vertebral body former of claim 2, wherein the outer tube (28) is sleeved on the exterior of the shape memory alloy tube (21), the outer tube (28) is formed integrally with the end cap (25) by injection molding; a top block (261) is sleeved between a distal end of the end cap (25) and the locking ring (26), and is in threaded connection with the distal end of the end cap (25); a proximal end of the locking ring (26) is fixed to the top block (261) through the end cap (25), the proximal end of the locking ring (26) can rotate about the end cap (25), and a distal end of the locking ring (26) is fixed with the sleeve handle (31) by the snap.

4. The multifunctional vertebral body former of claim 1, wherein a proximal end of the balloon handle (11) extends a protrusion (111) relative to a proximal end of the balloon handle fitting (12) and a proximal end of the balloon delivery snap (13), a proximal end surface of the protrusion (111) is closed to form a cavity, a side wall of the protrusion (111) is provided with a cavity interface (112); the balloon outer tube (16) is in communication with the outside through the protrusion (111).

5. The multifunctional vertebral body former of claim 4, wherein the outside of the proximal end surface of the protrusion (111) is connected to a supporting wire seat (113) through a screw thread, the supporting wire seat (113) is fixedly connected with a proximal end of the supporting wire (18), the supporting wire (18) is inserted into a pore channel (114) at the proximal end surface of the protrusion (111), the balloon outer tube (16) and the balloon (15) in sequence, and finally, a distal end surface of the supporting wire (18) is in contact with the distal end of the balloon (15).

6. The multifunctional vertebral body former of claim 5, wherein a balloon inner tube (17) is further provided or not provided between the balloon outer tube (16) and the supporting wire (18), a proximal end of the balloon inner tube (17) is fixed to the pore channel (114) on the proximal end surface of the protrusion (111), a distal end of the balloon inner tube (17) is fixedly connected with the distal end of the balloon (15).

7. The multifunctional vertebral body former of claim 6, wherein the balloon inner tube (17) is not provided, the balloon outer tube (16) is inserted from the proximal end of the balloon (15) into the distal end of the balloon (15), both the proximal end and the distal end of the balloon (15) are welded to the balloon outer tube (16) by sweat soldering or laser welding; an outer tube opening (161) is provided on the balloon outer tube (16) in an interior of the balloon (15); the balloon outer tube (16) at the distal end of the balloon (15) is sealed with UV adhesives (153) or PU rods (154) .

8. The multifunctional vertebral body former of claim 6, wherein the balloon inner tube (17) is provided, the distal end of the balloon (15) is welded to the balloon inner tube (17) through a transition tube (152) by sweat soldering or laser welding; after a proximal end of a plug (151) is welded to the distal end of the supporting wire (18) by laser welding, the proximal end of a plug (151) and the distal end of the balloon (15) are bonded to and sealed with medical grade glue.

9. The multifunctional vertebral body former of claim 1, wherein a reinforcing tube (121) is also sleeved on an outer surface of the balloon outer tube (16), and the reinforcing tube (121) is bonded to the balloon outer tube (16) with the medical grade glue, and the reinforcing tube (121) and the balloon handle fitting (12) are integrally injection molded.

10. The multifunctional vertebral body former of claim 1, wherein a section of a curved portion (27) is provided at the distal end of the shape memory alloy tube (21).

## Patentansprüche

1. Multifunktionaler Wirbelkörperformer, umfassend:
eine Ballonstruktur (1), die eine Ballonaußenröhre (16), einen Ballongriff (11) und einen Ballon (15) umfasst, wobei eine Außenfläche eines proximalen Endes der Ballonaußenröhre (16) in einer Abfolge von innen nach außen mit einem Ballongriffanschlussteil (12), dem Ballongriff (11) und einem Ballonzuführschnapper (13) versehen ist, wobei der Ballongriff (11) über einen Schnapper auf drehfest angebrachte Weise mit dem Ballonzuführschnapper (13) verbunden ist, das Ballongriffanschlussteil (12) fest mit dem Ballongriff (11) verbunden ist, ein distales Ende der Ballonaußenröhre (16) fest mit dem Ballon (15) verbunden ist und der der Ballon (15) an einem distalen Ende davon abgedichtet ist,
eine Transportröhrenstruktur (2), die eine Formgedächtnislegierungsröhre (21), einen Schieber (23) und einen Griff (22) zum Verstellen der Außenröhre umfasst, wobei die Formgedächtnislegierungsröhre (21) auf die Ballonaußenröhre (16) und eine Außenseite des Ballons (15) aufgeschoben ist, die Formgedächtnislegierungsröhre (21) mittels Spritzgießens integral mit dem Schieber (13) ausgebildet ist, ein proximales Ende des Schiebers (23) durch eine erste Klemmnase (231) lösbar mit dem Ballonzuführschnapper (13) verbunden ist, der Schieber (23) mit einer Gewindeaußenfläche versehen ist, wobei der Griff (22) zum Verstellen der Außenröhre mit einer Innengewindestruktur auf die Außenfläche des Schiebers (23) aufgeschoben ist, wobei der Griff (22) zum Verstellen der Außenröhre wirkgedreht wird, um den Schieber (23) anzutreiben, so dass dieser sich hin- und herbewegt, so dass ein Ausmaß, in dem ein distales Ende der Formgedächtnislegierungsröhre (21) bezüglich eines distalen Endes der Außenröhre (28) vorragt, und ein Ausmaß, in dem ein distales Ende des Ballons (15) bezüglich des distalen Endes der Formgedächtnislegierungsröhre (21) vorragt, verstellbar gesteuert werden.

2. Multifunktionaler Wirbelkörperformer nach Anspruch 1, wobei die Ballonstruktur (1) ferner ein Ballonzuführschnapper-Anschlussteil (14), das außerhalb des Ballonzuführschnappers (13) aufgeschoben ist, umfasst, eine zweite Klemmnase (131) an einem distalen Ende des Ballonzuführschnappers (13) in eine Gleitrutsche (141) einer Innenfläche des Ballonzuführschnapper-Anschlussteils (14) eingeführt ist, ein distales Ende der Gleitrutsche (141) mit einem Schlitz (142) versehen ist, ein distales Ende des Ballonzuführschnapper-Anschlussteils (14) über einen Schnapper lösbar mit einem Begrenzungsglied (24) verbunden ist, das außerhalb des Schiebers (23) aufgeschoben ist, sich ein abgerundeter Innenrand an einem distalen Ende des Begrenzungsglieds (24) über eine Vielzahl von bogenförmigen Abschnitten (242) erstreckt, die entlang einer Außenfläche des Schiebers (23) in einer Richtung zu dem distalen Ende nicht miteinander verbunden sind, ein distales Ende jedes der Vielzahl von bogenförmigen Abschnitten (242) über einen zylindrischen Stift (251) an einem proximalen Ende einer Endkappe (25) befestigt ist, die Endkappe (25) über einen Verriegelungsring (26) an einen Hülsengriff (31) gekoppelt und mit diesem verriegelt wird, der Griff (22) zum Verstellen der Außenröhre außerhalb der bogenförmigen Abschnitte (242) aufgeschoben ist und sich die Gewindestruktur an der Außenfläche des Schiebers (23) über einen Spalt der bogenförmigen Abschnitte (242) hinaus erstreckt und mit der Innengewindestruktur des Griffs (22) zum Verstellen der Außenröhre koppelt.

3. Multifunktionaler Wirbelkörperformer nach Anspruch 2, wobei die Außenröhre (28) auf das Äußere der Formgedächtnislegierungsröhre (21) aufgeschoben ist, die Außenröhre (28) mittels Spritzgießens integral mit der Endkappe (25) ausgebildet ist, ein oberer Block (261) zwischen einem distalen Ende der Endkappe (25) und dem Verriegelungsring (26) aufgeschoben ist und mit dem distalen Ende der Endkappe (25) in Gewindeverbindung steht, ein proximales Ende des Verriegelungsrings (26) durch die Endkappe (25) an dem oberen Block (261) befestigt ist, sich das proximale Ende des Verriegelungsrings (26) um die Endkappe (25) drehen kann und ein distales Ende des Verriegelungsrings (26) über den Schnapper mit dem Hülsengriff (31) befestigt ist.

4. Multifunktionaler Wirbelkörperformer nach Anspruch 1, wobei das proximale Ende des Ballongriffs (11) einen Vorsprung (111) bezüglich eines proximalen Endes des Ballongriffanschlussteils (12) und eines proximalen Endes des Ballonzuführschnappers (13) verlängert, eine proximale Endfläche des Vorsprungs (111) zum Bilden eines Hohlraums verschlossen ist, eine Seitenwand des Vorsprungs (111) mit einer Hohlraumschnittstelle (112) versehen ist und die Ballonaußenröhre (16) durch den Vorsprung (111) mit der Außenumgebung kommuniziert.

5. Multifunktionaler Wirbelkörperformer nach Anspruch 4, wobei die Außenseite der proximalen Endfläche des Vorsprungs (111) durch ein Schraubengewinde mit einem Stützdrahtsitz (113) verbunden ist, der Stützdrahtsitz (113) fest mit einem proximalen Ende des Stützdrahts (18) verbunden ist, der Stützdraht (18) in Abfolge in einen Porenkanal (114) an der proximalen Endfläche des Vorsprungs (111), die Ballonaußenröhre (16) und den Ballon (15) eingeführt ist und schließlich eine distale Endfläche des Stützdrahts (18) mit dem distalen Ende des Ballons (15) in Kontakt ist.

6. Multifunktionaler Wirbelkörperformer nach Anspruch 5, wobei eine Balloninnenröhre (17) ferner gegebenenfalls zwischen der Ballonaußenröhre (16) und dem Stützdraht (18) vorgesehen ist, ein proximales Ende der Balloninnenröhre (17) an der proximalen Endfläche des Vorsprungs (111) an dem Porenkanal (114) befestigt ist und ein distales Ende der Balloninnenröhre (17) fest mit dem distalen Ende des Ballons (15) verbunden ist.

7. Multifunktionaler Wirbelkörperformer nach Anspruch 6, wobei die Balloninnenröhre (17) nicht vorgesehen ist, die Ballonaußenröhre (16) von dem proximalen Ende des Ballons (15) in das distale Ende des Ballons (15) eingeführt wird, sowohl das proximale Ende als auch das distale Ende des Ballons (15) mittels Heißlötens oder Laserschweißens an die Ballonaußenröhre (16) angeschweißt werden, eine Außenröhrenöffnung (161) an der Ballonaußenröhre (16) in einem Inneren des Ballons (15) vorgesehen ist und die Ballonaußenröhre (16) an dem distalen Ende des Ballons (15) mit UV-Klebstoffen (153) oder PU-Stäben (154) versiegelt ist.

8. Multifunktionaler Wirbelkörperformer nach Anspruch 6, wobei die Balloninnenröhre (17) vorgesehen ist, das distale Ende des Ballons (15) durch eine Übergangsröhre (152) mittels Heißlötens oder Laserschweißens an die Balloninnenröhre (17) geschweißt ist und das proximale Ende eines Stopfens (151) und das distale Ende des Ballons (15) mit medizinischem Klebstoff verklebt und versiegelt sind, nachdem ein proximales Ende eines Stopfens (151) mittels Laserschweißens an das distale Ende des Stützdrahts (18) geschweißt worden ist.

9. Multifunktionaler Wirbelkörperformer nach Anspruch 1, wobei eine Verstärkungsröhre (121) ebenfalls über eine Außenfläche der Ballonaußenröhre (16) geschoben ist und die Verstärkungsröhre (121) mit dem medizinischen Klebstoff an die Ballonaußenröhre (16) geklebt ist und die Verstärkungsröhre (121) und das Ballongriffanschlussteil (12) integral spritzgegossen sind.

10. Multifunktionaler Wirbelkörperformer nach Anspruch 1, wobei eine Sektion eines gekrümmten Abschnitts (27) an dem distalen Ende der Formgedächtnislegierungsröhre (21) vorgesehen ist.

## Revendications

1. Dispositif de formation de corps vertébral multifonctionnel, comprenant :
une structure de ballonnet (1) comprenant un tube externe de ballonnet (16), une poignée de ballonnet (11) et un ballonnet (15) ; une surface externe d'une extrémité proximale du tube externe de ballonnet (16) est pourvue, dans un ordre allant de l'intérieur vers l'extérieur, d'un raccord de poignée de ballonnet (12), de la poignée de ballonnet (11) et d'un élément à déclic de pose de ballonnet (13) ; dans lequel la poignée de ballonnet (11) est reliée à l'élément à déclic de pose de ballonnet (13) par un raccord à déclic d'une manière fixe en rotation, le raccord de poignée de ballonnet (12) est relié de manière fixe à la poignée de ballonnet (11) ; une extrémité distale du tube externe de ballonnet (16) est reliée de manière fixe au ballonnet (15), le ballonnet (15) est scellé à une extrémité distale de celui-ci ;
une structure de tube de transport (2), comprenant un tube en alliage à mémoire de forme (21), un curseur (23), et une poignée de réglage de tube externe (22), dans laquelle le tube en alliage à mémoire de forme (21) est emmanché sur le tube externe de ballonnet (16) et un extérieur du ballonnet (15), le tube en alliage à mémoire de forme (21) est formé d'un seul tenant avec le curseur (13) par moulage par injection ; une extrémité proximale du curseur (23) est reliée de manière amovible à l'élément à déclic de pose de ballonnet (13) par l'intermédiaire d'une première patte de serrage (231) ; le curseur (23) est pourvu d'une surface externe filetée, la poignée de réglage de tube externe (22) ayant une structure de filet interne est emmanchée sur la surface externe du curseur (23), dans lequel la poignée de réglage de tube externe (22) est tournée de manière opérationnelle pour entraîner un mouvement d'avant en arrière du curseur (23), de sorte qu'une quantité saillante d'une extrémité distale du tube en alliage à mémoire de forme (21) par rapport à une extrémité distale du tube externe (28), et qu'une quantité saillante d'une extrémité distale du ballonnet (15) par rapport à l'extrémité distale du tube en alliage à mémoire de forme (21) soient contrôlées de manière réglable.

2. Dispositif de formation de corps vertébral multifonctionnel selon la revendication 1, dans lequel la structure de ballonnet (1) comprend en outre un raccord d'élément à déclic de pose de ballonnet (14) emmanché à l'extérieur de l'élément à déclic de pose de ballonnet (13), une seconde patte de serrage (131) au niveau d'une extrémité distale de l'élément à déclic de pose de ballonnet (13) est insérée dans une goulotte coulissante (141) d'une surface interne du raccord d'élément à déclic de pose de ballonnet (14), une extrémité distale de la goulotte coulissante (141) est pourvue d'une fente (142) ; une extrémité distale du raccord d'élément à déclic de pose de ballonnet (14) est reliée de manière amovible à un élément de limitation (24) emmanché à l'extérieur du curseur (23) par un élément à déclic ; un bord interne arrondi au niveau d'une extrémité distale de l'élément de limitation (24) étend une pluralité de parties en forme d'arc (242) qui ne sont pas reliées entre elles le long d'une surface externe du curseur (23) dans une direction vers l'extrémité distale, une extrémité distale de chaque partie de la pluralité de parties en forme d'arc (242) est fixée à une extrémité proximale d'un capuchon d'extrémité (25) par une broche cylindrique (251), le capuchon d'extrémité (25) correspond à une poignée de manchon (31) et est verrouillé à celle-ci par un anneau de verrouillage (26) ; la poignée de réglage de tube externe (22) est emmanchée à l'extérieur des parties en forme d'arc (242), la structure filetée sur la surface externe du curseur (23) s'étend au-delà d'un espace des parties en forme d'arc (242), et correspond à la structure filetée interne de la poignée de réglage de tube externe (22).

3. Dispositif de formation de corps vertébral multifonctionnel selon la revendication 2, dans lequel le tube externe (28) est emmanché sur l'extérieur du tube en alliage à mémoire de forme (21), le tube externe (28) est formé d'un seul tenant avec le capuchon d'extrémité (25) par moulage par injection ; un bloc supérieur (261) est emmanché entre une extrémité distale du capuchon d'extrémité (25) et l'anneau de verrouillage (26), et est en liaison filetée avec l'extrémité distale du capuchon d'extrémité (25) ; une extrémité proximale de l'anneau de verrouillage (26) est fixée au bloc supérieur (261) à travers le capuchon d'extrémité (25), l'extrémité proximale de l'anneau de verrouillage (26) peut tourner autour du capuchon d'extrémité (25), et une extrémité distale de l'anneau de verrouillage (26) est fixée à la poignée de manchon (31) par le raccord à déclic.

4. Dispositif de formation de corps vertébral multifonctionnel selon la revendication 1, dans lequel une extrémité proximale de la poignée de ballonnet (11) étend une protubérance (111) par rapport à une extrémité proximale du raccord de poignée de ballonnet (12) et à une extrémité proximale de l'élément à déclic de pose de ballonnet (13), une surface d'extrémité proximale de la protubérance (111) est fermée pour former une cavité, une paroi latérale de la protubérance (111) est pourvue d'une interface de cavité (112) ; le tube externe de ballonnet (16) est en communication avec l'extérieur par l'intermédiaire de la protubérance (111).

5. Dispositif de formation de corps vertébral multifonctionnel selon la revendication 4, dans lequel l'extérieur de la surface d'extrémité proximale de la protubérance (111) est relié à un siège de fil de support (113) par l'intermédiaire d'un pas de vis, le siège de fil de support (113) est relié de manière fixe à une extrémité proximale du fil de support (18), le fil de support (18) est inséré dans un canal de pore (114) à la surface d'extrémité proximale de la protubérance (111), le tube externe de ballonnet (16) et le ballonnet (15) dans cet ordre, et enfin, une surface d'extrémité distale du fil de support (18) est en contact avec l'extrémité distale du ballonnet (15).

6. Dispositif de formation de corps vertébral multifonctionnel selon la revendication 5, dans lequel un tube interne de ballonnet (17) est en outre fourni ou non fourni entre le tube externe de ballonnet (16) et le fil de support (18), une extrémité proximale du tube interne de ballonnet (17) est fixée au canal de pore (114) sur la surface d'extrémité proximale de la protubérance (111), une extrémité distale du tube interne de ballonnet (17) est reliée de manière fixe à l'extrémité distale du ballonnet (15).

7. Dispositif de formation de corps vertébral multifonctionnel selon la revendication 6, dans lequel le tube interne de ballonnet (17) n'est pas fourni, le tube externe de ballonnet (16) est inséré depuis l'extrémité proximale du ballonnet (15) dans l'extrémité distale du ballonnet (15), l'extrémité proximale et l'extrémité distale du ballonnet (15) sont toutes deux soudées au tube externe de ballonnet (16) par brasage tendre avec préenrobage des bords ou par soudage laser ; une ouverture de tube externe (161) est fournie sur le tube externe de ballonnet (16) dans un intérieur du ballonnet (15) ; le tube externe de ballonnet (16) au niveau de l'extrémité distale du ballonnet (15) est scellé à l'aide d'adhésifs UV (153) ou de tiges en PU (154).

8. Dispositif de formation de corps vertébral multifonctionnel selon la revendication 6, dans lequel le tube interne de ballonnet (17) est fourni, l'extrémité distale du ballonnet (15) est soudée au tube interne de ballonnet (17) à travers un tube de transition (152) par brasage tendre avec préenrobage des bords ou soudage laser ; après qu'une extrémité proximale d'un bouchon (151) a été soudée à l'extrémité distale du fil de support (18) par soudage laser, l'extrémité proximale d'un bouchon (151) et l'extrémité distale du ballonnet (15) sont liées et scellées avec une colle de qualité médicale.

9. Dispositif de formation de corps vertébral multifonctionnel selon la revendication 1, dans lequel un tube de renforcement (121) est également emmanché sur une surface externe du tube externe de ballonnet (16), et le tube de renforcement (121) est lié au tube externe de ballonnet (16) avec la colle de qualité médicale, et le tube de renforcement (121) et le raccord de poignée de ballonnet (12) sont moulés par injection d'un seul tenant.

10. Dispositif de formation de corps vertébral multifonctionnel selon la revendication 1, dans lequel une section d'une partie incurvée (27) est fournie au niveau de l'extrémité distale du tube en alliage à mémoire de forme (21).
